# EUROPEAN PATENT SPECIFICATION

(11) **EP 2 731 925 B1**
(45) Date of publication and mention of the grant of the patent: **08.07.2015**
(21) Application number: 12766790.5
(22) Date of filing: 14.09.2012
(51) Int. Cl.: C07C 51/62, C07C 201/12, C07C 63/307, C07C 53/42, C07C 205/57

(54) **IN-SITU METHOD FOR PREPARING HYDROLYZED ACYL HALIDE COMPOUND**
IN-SITU-VERFAHREN ZUR HERSTELLUNG EINER HYDROLYSIERTEN ACYLHALOGENVERBINDUNG
PROCÉDÉ DE PRÉPARATION IN SITU D'UN COMPOSÉ D'HALOGÉNURE D'ACYLE HYDROLYSÉ

(30) Priority: 29.09.2011 US 201161540551 P
(43) Date of publication of application: 21.05.2014
(73) Proprietor: Dow Global Technologies LLC, Midland, MI 48674 (US)
(72) Inventor: ROY, Abhishek, Edina, MN 55439 (US); JONS, Steven, D., Eden Prairie, MN 55346 (US); KOOB, Joseph, D., Midland, MI 48674 (US); PEERY, Martin, H., Bloomington, MN 55438 (US); QIU, XiaoHua, Sam, Midland, MI 48642 (US); ROSENBERG, Steven, Shorewood, MN 55331 (US); TOMLINSON, Ian, A., Midland, MI 48642 (US)
(74) Representative: Houghton, Mark Phillip
(86) International application number: PCT/US2012/055264
(87) International publication number: WO 2013/048762

(56) References cited:
- US-A- 5 736 371
- C. S. MARVEL ET AL: THE JOURNAL OF ORGANIC CHEMISTRY, vol. 18, no. 12, 1 December 1953 (1953-12-01), pages 1664-1669, XP055045070, ISSN: 0022-3263, DOI: 10.1021/jo50018a007

## Description

### FIELD OF THE INVENTION:

The present invention is directed toward in-situ methods for preparing a variety of hydrolyzed acyl halide compounds in a hydrocarbon or halogenated hydrocarbon solution.

### BACKGROUND:

While acyl halide compounds such as benzene-1,3,5-tricarboyl trichloride and adipoyl dichloride are readily soluble in a variety of hydrocarbon and halogenated hydrocarbon solvents, their hydrolyzed and partially hydrolyzed analogs (3,5-bis(chlorocarbonyl) benzoic acid and 6-chloro-6-oxohexanoic acid are only slightly soluble, e.g. less than 0.02 wt. %. As a consequence, it is difficult to prepare in-situ solutions of such hydrolyzed reaction products in a hydrocarbon or halogenated hydrocarbon solvent. Nevertheless, hydrocarbon and halogenated hydrocarbon solutions including such hydrolyzed reaction products would be useful in a variety of applications including the preparation of polyamides. US 5736371 describes a process for preparing succinic acid monochloride. Marvel, et al., Journal of Organic Chemistry, vol 18, no.12, (1953) 1664-1669 describes a process for preparing p-chloroformylbenzoic acid.

### BRIEF SUMMARY OF THE INVENTION:

The present invention includes a method for preparing a hydrocarbon reaction product comprising at least one acyl halide functional group and a carboxylic acid functional group in a solution comprising a hydrocarbon or halogenated hydrocarbon solvent, wherein the reaction product has a solubility limit of less than 1 weight percent in the solvent and is produced to a molar concentration greater than its solubility limit within the solvent while remaining soluble in solution. The solution comprises at least 80 v/v% of the solvent in combination with: i) water at a molar concentration greater than its solubility limit within the solvent but less that its solubility limit with the solution, ii) a reactant comprising a plurality of acyl halide functional groups at a molar concentration less than its solubility limit within the solvent and at a molar ratio with water from 1:2 to 1000:1, and iii) a tri-hydrocarbyl phosphate compound at a molar ratio with the hydrocarbon reactant from 100:1 to 1:1000.

While many different embodiments are described, preferred embodiments provide a method for preparing a hydrolyzed or partially hydrolyzed acyl halide compound at a concentration above its solubility limit within the corresponding hydrocarbon or halogenated hydrocarbon solvent.

### DETAILED DESCRIPTION OF THE INVENTION:

The present invention broadly includes in-situ methods for preparing reaction products in a solution comprising: a reactant, hydrocarbon or halogenated hydrocarbon solvent, water (preferably deionized) and a tri-hydrocarbyl phosphate compound.

The reactant(s) of the present invention includes an aliphatic or arene moiety including a plurality of acyl halide functional groups. The reaction product comprises a hydrolyzed, preferably mono-hydrolyzed, analog of the reactant.

In a preferred set of embodiments, the reactant and reaction product have molecular weights less than 700, 600, 500, 400 or 300 unified atomic mass units, "u" (Daltons). In another set of embodiments, the reactant and reaction product comprises equal to or less than 30, 20, 15 or 12 carbon atoms, and preferably include more than 3 carbon atoms. In yet another set of embodiments, the reactant and reaction product comprises from 4 to 12 carbon atoms. Non-limiting examples of reactants based upon aliphatic moieties include: C₄ through C₁₂ alkanes (e.g. succinyl, glutaroyl, adipoyl, heptanedioyl, octanedioyl, nonanedioyl, decanedioyl, undecanedioyl and dodecanedioyl di and tri chloride), cycloalkanes (e.g. cyclopropane tri carboxylic acid chloride, cyclobutane tetra carboxylic acid chloride, cyclopentane tri carboxylic acid chloride, cyclopentane tetra carboxylic acid chloride, cyclohexane tri carboxylic acid chloride, tetrahydrofuran tetra carboxylic acid chloride, cyclopentane dicarboxylic acid chloride, cyclobutane dicarboxylic acid chloride, cyclohexane di carboxylic acid chloride, tetrahydrofuran dicarboxylic acid chloride, cyclohexane dichloride, cyclohexane-1,3,5-tricarbonyl trichloride, and decahydronaphthalene-2,6-dicarbonyl dichloride. Non-limiting examples of reactants based upon arene moieties include: terephthaloyl dichloride, isophthalic acid chloride, benzene-1,3,5-tricarbonyl trichloride and naphthalene-2,6-dicarbonyl dichloride. Additional examples of reactants include branched analogs of the preceding compounds along analogs including additional acyl halide functional groups. Examples of preferred reaction products include the mono-hydrolyzed analog of the preceding compounds.

The selection of hydrocarbon or halogenated hydrocarbon solvent is not particularly limited and combinations of multiple solvents may be used. The solvent is preferably a liquid at 20°C (101 kPa). The solvent preferably has a water solubility of less than 800 ppm (and more preferably less than 500, 400, 300, or 200, or in some embodiments, less than 150 ppm). As used herein, the term "water solubility" refers to the concentration of water that is soluble in a chosen hydrocarbon solvent measured at 20°C (101 kPa) as measured by ASTM D4928-11. Non-limiting examples of applicable hydrocarbon solvents include: paraffins (e.g. hexane, cyclohexane, heptane, octane, dodecane), isoparaffins (e.g. ISOPAR™ L), aromatics (e.g. benzene, 1,3,5-trimethylbenzene, toluene,) and halogenated hydrocarbons (e.g. FREON™ series, chlorobenzene, di and trichlorobenzene).

Tri-hydrocarbyl phosphate compounds applicable in the present invention are those represented by Formula (I): 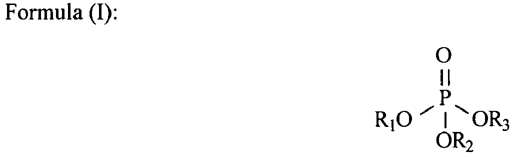 wherein "P" is phosphorous, "O" is oxygen and R₁, R₂ and R₃ are independently selected from hydrogen and hydrocarbyl groups comprising from 1 to 10 carbon atoms, with the proviso that no more than one of R₁, R₂ and R₃ are hydrogen. R₁, R₂ and R₃ are preferably independently selected from aliphatic and arene groups. Applicable aliphatic groups include both branched and unbranched species, e.g. methyl, ethyl, propyl, isopropyl, butyl, isobutyl, pentyl, 2-pentyl, 3-pentyl, cyclopentyl, cyclohexyl, etc.; however, alkyl groups having from 3 to 10 carbon atoms are preferred. Applicable arene groups include phenyl and naphthyl groups. Specific examples of tri-hydrocarbyl phosphate compounds include: tripropyl phosphate, tributyl phosphate, tripentyl phosphate, trihexyl phosphate, triphenyl phosphate, propyl biphenyl phosphate, dibutyl phenyl phosphate, butyl diethyl phosphate, dibutyl hydrogen phosphate, butyl heptyl hydrogen phosphate and butyl heptyl hexyl phosphate.

The aforementioned constituents are combined to form a solution comprising at least 80 v/v% solvent, and in some embodiments at least 90 v/v%, 92 v/v% or 95 v/v% solvent along with: i) water at a molar concentration greater than its solubility limit (i.e. miscibility limit) within the solvent but less that its solubility limit with the solution, ii) the reactant at a molar concentration less than its solubility limit within the solvent and at a molar ratio with water from 1:2 to 1000:1, and iii) the tri-hydrocarbyl phosphate compound at a molar ratio with the hydrocarbon reactant from 100:1 to 1:1000. In a preferred embodiment, the solution comprises the tri-hydrocarbyl phosphate compound at a molar ratio with the reactant from 10:1 to 1:100. In another embodiment, the solution comprises the reactant at a molar ratio with water of from 1:2 to 200:1, and in other embodiments from 1:2 to 100:1. In still another embodiment, the solution includes at least one but preferably all of the following: water at a concentration of less than 1 wt%, the reactant at a concentration of less than 10 wt% or the tri-hydrocarbyl phosphate compound at a concentration of less than 10 wt%. In yet other set of embodiments, the solution includes at least one but preferably all of the following: water at a concentration of less than 0.5 wt%, the reactant at a concentration of less than 5 wt%, or the tri-hydrocarbyl phosphate compound at a concentration of less than 5 wt%.

The aforementioned constituents may be combined and mixed within a reaction vessel at room temperature. While the order of addition is not particularly limited, in preferred embodiments the reactant is contacted with the tri-hydrocarbyl phosphate compound prior to contact with water. The resulting reaction product is the hydrolyzed analog of the reactant. In preferred embodiments, the mono-hydrolyzed analog of the reactant is the dominant reaction product, e.g. preferably at least 60 wt. %, at least 70 wt. %, at least 80 wt. % or still more preferably at least 90 wt%. Representative reaction pathways are illustrated below.

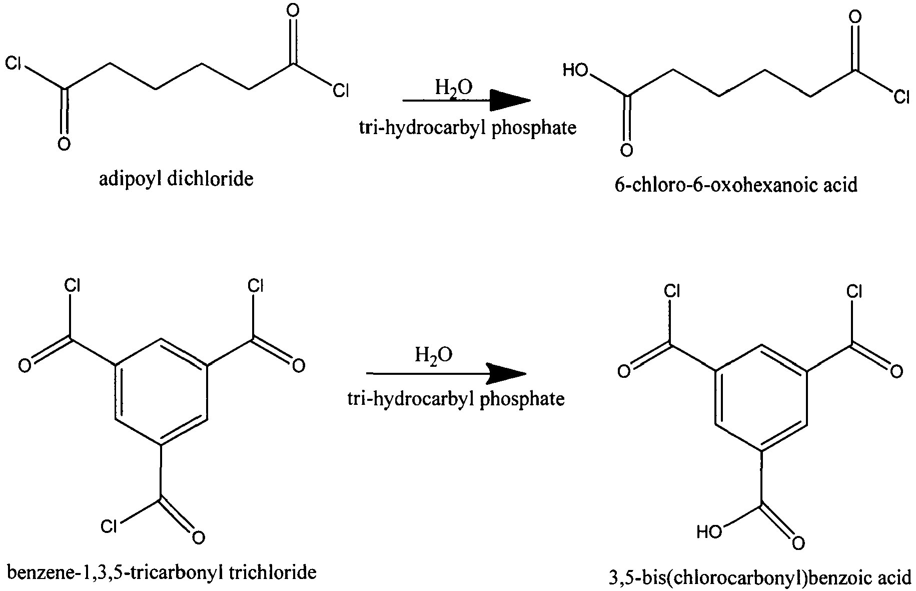

The reaction product has a solubility limit of less than 1 wt. % in the solvent, and in some embodiments less than 0.1 wt%, 0.05 wt % and still others less than even 0.02 wt%. While the reaction product is produced to a molar concentration greater than its solubility limit (e.g. greater than 10%) within the solvent, the product remains soluble in solution. While not wishing to be bound by theory, it is believed that the subject class of tri-hydrocarbyl phosphates increases the solubility of water (and hydrolyzed reaction product) within the hydrocarbon solvent and facilitates hydrolysis of the reactant. Surprisingly, the reaction product is highly selective toward the mono-hydrolyzed form. Hydrocarbon-based solutions including relatively higher concentrations of the hydrolyzed reaction product are useful in a variety of applications. In one application, solutions including both the reactant and hydrolyzed product (particularly mono-hydrolyzed product) are useful in coating applications to prepare polyamides.

Many embodiments of the invention have been described and in some instances certain embodiments, selections, ranges, constituents, or other features have been characterized as being "preferred." Characterizations of "preferred" features should in no way be interpreted as deeming such features as being required, essential or critical to the invention. For purposes of this description, the terms "acyl halide" and "acid halide" have the same meaning. While much of the description has focused upon acyl chlorides, non-chloride halides are also included. The term "solubility limit" refers to the point at which no additional amount of a constituent, (e.g. water, reaction product, reactant) is miscible or dissolvable with the hydrocarbon solvent or solution, as measured at 20°C and 101 kPa. Unless otherwise stated, all solubility related parameters are determined at 20°C and 101 kPa.

### EXAMPLES

### In situ preparation of mono-hydrolyzed polyfunctional acid chlorides:

A solution of trialkylphosphate in a hydrocarbon solvent was combined with trace water and stirred vigorously at room temperature (reactant concentrations are provided in Table 1 below). The reaction was monitored by proton NMR. Over time the water is consumed and the composition of product reaction mixture becomes constant. If the desired amount of hydrolysis was not obtained, a second addition of water was introduced to the reaction mixture (noted as a "+" quantity in the water concentration column in Table 1). The solution was allowed to stir until the product reaction mixture remained constant as observed by ¹H NMR and the final product reaction mixture is noted in Table 1.

**Table 1:**

| Ex. No. | Acid Chloride¹ | | Trialkyl-phosphate² | | water conc. (ppm) | solvent | reaction time (min) | product reaction mixture (mole acid chloride: mole mono-hydrolyzed acid chloride) |
|---|---|---|---|---|---|---|---|---|
| | type | conc. (wt%) | type | conc. (wt%) | | | | |
| 1-1 | TMC | 0.29 | TBP | 1.1 | 99 | Isopar L | 10 | 59:41 |
| 1-2 | IPC | 1.8 | TBP | 0.47 | 50+100 | Isopar L | 1200 | 88:12 |
| 1-3 | NO2IPC | 0.26 | TBP | 0.29 | 50 | Isopar L | 400 | 80:20 |
| 1-4 | TMC | 2.3 | TBP | 1.0 | 50 + 200 | 90 Isopar L: 10 toluene | 120 | 70:30 |
| 1-5 | TMC | 0.3 | TEP | 1.0 | 99 | Isopar L | 5 | 85:15 |
| 1-6 | TMC | 0.32 | TEHP | 1.0 | 99 | Isopar L | 60 | 69:31 |

| | | | | | | | | |
|---|---|---|---|---|---|---|---|---|
| ¹trimesoyl chloride (TMC); isophthaloyl chloride (IPC); 5-nitroisophthaloyl chloride (NO2IPC) ²tributylphosphate (TBP); triethylphosphate (TEP); triethylhexylphophate (TEHP) | | | | | | | | |

## Claims

1. A method for preparing a reaction product comprising at least one acyl halide functional group and a carboxylic acid functional group in a solution comprising a hydrocarbon or halogenated hydrocarbon solvent having a water solubility of less than 800 ppm;
wherein the reaction product has a solubility limit of less than 1 weight percent in the solvent and is produced to a molar concentration greater than its solubility limit within the solvent while remaining soluble in solution, and
wherein the solution comprises at least 80 v/v% solvent in combination with:
i) water at a molar concentration greater than its solubility limit within the solvent but less that its solubility limit with the solution,
ii) a reactant comprising a plurality of acyl halide functional groups at a molar concentration less than its solubility limit within the solvent and at a molar ratio with water from 1:2 to 1000:1, and
iii) a tri-hydrocarbyl phosphate compound at a molar ratio with the hydrocarbon reactant from 100:1 to 1:1000, wherein the tri-hydrocarbyl phosphate compound is represented by: 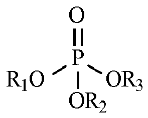 wherein R₁, R₂ and R₃ are independently selected from hydrogen and hydrocarbyl groups comprising from 1 to 10 carbon atoms, with the proviso that no more than one of R₁, R₂ and R₃ are hydrogen.

2. The method of claim 1 wherein the reactant and reaction product both have a molecule weight less than 700 u (Daltons).

3. The method of claim 1 wherein the reactant and reaction product both have a molecule weight less than 300 u (Daltons).

4. The method of claim 1 wherein the reactant and reaction product both comprise equal to or less than 30 carbon atoms.

5. The method of claim 1 wherein the reactant and reaction product both comprise from 4 to 12 carbon atoms

6. The method of claim 1 wherein the reactant comprises a carbon containing moiety selected from: an aliphatic or arene group substituted with a plurality of acyl halide functional groups; and the reaction product comprises an aliphatic or arene group substituted with at least one acyl halide functional group and a carboxylic acid functional group.

7. The method of claim 1 wherein the reaction product comprises an aliphatic or arene group substituted with at least one acyl halide functional group and single carboxylic acid functional group.

8. The method of claim 1 wherein the solution comprises:
i) water at a concentration of less than 1 wt%,
ii) the reactant at a concentration of less than 10 wt% and
iii) the tri-hydrocarbyl phosphate compound at a concentration of less than 10 wt%.

9. The method of claim 1 wherein the solution comprises:
i) water at a concentration of less than 0.5 wt%,
ii) the reactant at a concentration of less than 5 wt%, and
iii) the tri-hydrocarbyl phosphate compound at a concentration of less than 5 wt%.

10. The method of claim 1 wherein the solution comprises the reactant at a molar ratio with water from 1:2 to 100:1.

11. The method of claim 1 wherein the solution comprises the tri-hydrocarbyl phosphate compound at a molar ratio with the reactant of 10:1 to 1:100.

12. The method of claim 1 wherein the solvent has a water solubility of less than 150 ppm.

13. The method of claim 1 wherein R₁, R₂ and R₃ are independently selected from: aliphatic and arene groups.

14. The method of claim 1 wherein R₁, R₂ and R₃ are independently selected from alkyl groups.

## Patentansprüche

1. Ein Verfahren zur Zubereitung eines Reaktionsproduktes, beinhaltend mindestens eine funktionelle Acylhalogenidgruppe und eine funktionelle Carbonsäuregruppe in einer Lösung, beinhaltend ein Kohlenwasserstoff- oder halogeniertes Kohlenwasserstofflösungsmittel mit einer Wasserlöslichkeit von weniger als 800 ppm; wobei das Reaktionsprodukt eine Löslichkeitsgrenze von weniger als 1 Gewichtsprozent in dem Lösungsmittel aufweist und mit einer Molkonzentration von mehr als seiner Löslichkeitsgrenze innerhalb des Lösungsmittels hergestellt wird, während es in Lösung löslich bleibt, und
wobei die Lösung zu mindestens 80 Volumenprozent Lösungsmittel beinhaltet, in Kombination mit:
i) Wasser mit einer Molkonzentration von mehr als seiner Löslichkeitsgrenze innerhalb des Lösungsmittels aber weniger als seiner Löslichkeitsgrenze innerhalb der Lösung,
ii) einem Reaktanten, beinhaltend eine Vielzahl von funktionellen Acylhalogenidgruppen mit einer Molkonzentration von weniger als seiner Löslichkeitsgrenze innerhalb des Lösungsmittels und mit einem Molverhältnis zu Wasser von 1 : 2 bis 1000 : 1, und
iii) einer Trihydrocarbylphosphatverbindung mit einem Molverhältnis zu dem Kohlenwasserstoffreaktanten von 100 : 1 bis 1 : 1000, wobei die Trihydrocarbylphosphatverbindung durch Folgendes dargestellt ist: 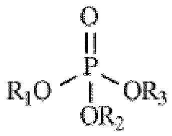
wobei R₁, R₂ und R₃ unabhängig ausgewählt sind aus Wasserstoff und Hydrocarbylgruppen, beinhaltend von 1 bis 10 Kohlenstoffatome, mit der Maßgabe, dass nicht mehr als einer von R₁, R₂ und R₃ Wasserstoff ist.

2. Verfahren gemäß Anspruch 1, wobei der Reaktant und das Reaktionsprodukt jeweils eine Molekülmasse von weniger als 700 u (Dalton) aufweisen.

3. Verfahren gemäß Anspruch 1, wobei der Reaktant und das Reaktionsprodukt jeweils eine Molekülmasse von weniger als 300 u (Dalton) aufweisen.

4. Verfahren gemäß Anspruch 1, wobei der Reaktant und das Reaktionsprodukt jeweils weniger als oder gleich 30 Kohlenstoffatome beinhalten.

5. Verfahren gemäß Anspruch 1, wobei der Reaktant und das Reaktionsprodukt jeweils von 4 bis 12 Kohlenstoffatome beinhalten.

6. Verfahren gemäß Anspruch 1, wobei der Reaktant einen Kohlenstoff enthaltenden Anteil beinhaltet, der aus Folgendem ausgewählt ist: einer aliphatischen Gruppe oder Arengruppe, substituiert mit einer Vielzahl von funktionellen Acylhalogenidgruppen; und wobei das Reaktionsprodukt eine aliphatische Gruppe oder Arengruppe beinhaltet, substituiert mit mindestens einer funktionellen Acylhalogenidgruppe und einer funktionellen Carbonsäuregruppe.

7. Verfahren gemäß Anspruch 1, wobei das Reaktionsprodukt eine aliphatische Gruppe oder Arengruppe beinhaltet, substituiert mit mindestens einer funktionellen Acylhalogenidgruppe und einer einzelnen funktionellen Carbonsäuregruppe.

8. Verfahren gemäß Anspruch 1, wobei die Lösung Folgendes beinhaltet:
i) Wasser mit einer Konzentration von weniger als 1 Gew.-%,
ii) den Reaktanten mit einer Konzentration von weniger als 10 Gew.-% und
iii) die Trihydrocarbylphosphatverbindung mit einer Konzentration von weniger als 10 Gew.-%.

9. Verfahren gemäß Anspruch 1, wobei die Lösung Folgendes beinhaltet:
i) Wasser mit einer Konzentration von weniger als 0,5 Gew.-%,
ii) den Reaktanten mit einer Konzentration von weniger als 5 Gew.-% und
iii) die Trihydrocarbylphosphatverbindung mit einer Konzentration von weniger als 5 Gew.-%.

10. Verfahren gemäß Anspruch 1, wobei die Lösung den Reaktanten mit einem Molverhältnis zu Wasser von 1 : 2 bis 100 : 1 beinhaltet.

11. Verfahren gemäß Anspruch 1, wobei die Lösung die Trihydrocarbylphosphatverbindung mit einem Molverhältnis zu dem Reaktanten von 10 : 1 bis 1 : 100 beinhaltet.

12. Verfahren gemäß Anspruch 1, wobei das Lösungsmittel eine Wasserlöslichkeit von weniger als 150 ppm aufweist.

13. Verfahren gemäß Anspruch 1, wobei R₁ R₂ und R₃ unabhängig aus Folgendem ausgewählt sind: aliphatischen Gruppen und Arengruppen.

14. Verfahren gemäß Anspruch 1, wobei R₁, R₂ und R₃ unabhängig ausgewählt sind aus Alkylgruppen.

## Revendications

1. Une méthode pour préparer un produit de réaction comprenant au moins un groupe fonctionnel halogénure d'acyle et un groupe fonctionnel acide carboxylique dans une solution comprenant un solvant hydrocarbure ou hydrocarbure halogéné ayant une solubilité de l'eau de moins de 800 ppm ;
dans laquelle le produit de réaction a une limite de solubilité de moins de 1 pour cent en poids dans le solvant et est produit jusqu'à une concentration molaire supérieure à sa limite de solubilité au sein du solvant tout en restant soluble en solution, et
dans laquelle la solution comprend au moins 80 % en volume par volume de solvant en combinaison avec :
i) de l'eau à une concentration molaire supérieure à sa limite de solubilité au sein du solvant mais inférieure à sa limite de solubilité avec la solution,
ii) un réactif comprenant une pluralité de groupes fonctionnels halogénure d'acyle à une concentration molaire inférieure à sa limite de solubilité au sein du solvant et à un rapport molaire avec l'eau allant de 1/2 à 1 000/1, et
iii) un composé phosphate de tri-hydrocarbyle à un rapport molaire avec le réactif hydrocarbure allant de 100/1 à 1/1 000, le composé phosphate de tri-hydrocarbyle étant représenté par : 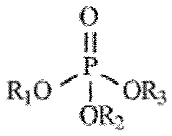 dans laquelle R₁, R₂ et R₃ sont indépendamment sélectionnés parmi l'hydrogène et des groupes hydrocarbyle comprenant de 1 à 10 atomes de carbone, à la condition que pas plus d'un R parmi R₁, R₂ et R₃ ne soit l'hydrogène.

2. La méthode de la revendication 1 dans laquelle le réactif et le produit de réaction ont tous deux une masse moléculaire inférieure à 700 u (Daltons).

3. La méthode de la revendication 1 dans laquelle le réactif et le produit de réaction ont tous deux une masse moléculaire inférieure à 300 u (Daltons).

4. La méthode de la revendication 1 dans laquelle le réactif et le produit de réaction comprennent tous deux un nombre d'atomes de carbone égal ou inférieur à 30.

5. La méthode de la revendication 1 dans laquelle le réactif et le produit de réaction comprennent tous deux de 4 à 12 atomes de carbone.

6. La méthode de la revendication 1 dans laquelle le réactif comprend un groupement contenant du carbone sélectionné parmi : un groupe aliphatique ou arène substitué par une pluralité de groupes fonctionnels halogénure d'acyle ; et le produit de réaction comprend un groupe aliphatique ou arène substitué par au moins un groupe fonctionnel halogénure d'acyle et un groupe fonctionnel acide carboxylique.

7. La méthode de la revendication 1 dans laquelle le produit de réaction comprend un groupe aliphatique ou arène substitué par au moins un groupe fonctionnel halogénure d'acyle et un groupe fonctionnel acide carboxylique unique.

8. La méthode de la revendication 1 dans laquelle la solution comprend :
i) de l'eau à une concentration de moins de 1 % en poids,
ii) le réactif à une concentration de moins de 10 % en poids et
iii) le composé phosphate de tri-hydrocarbyle à une concentration de moins de 10 % en poids.

9. La méthode de la revendication 1 dans laquelle la solution comprend :
i) de l'eau à une concentration de moins de 0,5 % en poids,
ii) le réactif à une concentration de moins de 5 % en poids, et
iii) le composé phosphate de tri-hydrocarbyle à une concentration de moins de 5 % en poids.

10. La méthode de la revendication 1 dans laquelle la solution comprend le réactif à un rapport molaire avec l'eau allant de 1/2 à 100/1.

11. La méthode de la revendication 1 dans laquelle la solution comprend le composé phosphate de tri-hydrocarbyle à un rapport molaire avec le réactif de 10/1 à 1/100.

12. La méthode de la revendication 1 dans laquelle le solvant a une solubilité de l'eau de moins de 150 ppm.

13. La méthode de la revendication 1 dans laquelle R₁, R₂ et R₃ sont indépendamment sélectionnés parmi : des groupes aliphatiques et arène.

14. La méthode de la revendication 1 dans laquelle R₁, R₂ et R₃ sont indépendamment sélectionnés parmi des groupes alkyle.
